# EUROPEAN PATENT SPECIFICATION

(11) **EP 2 300 516 B1**
(45) Date of publication and mention of the grant of the patent: **03.10.2012**
(21) Application number: 09785329.5
(22) Date of filing: 14.07.2009
(51) Int. Cl.: C08J 3/20, C08J 9/26

(54) **POLYMERIC MATERIALS**
POLYMERMATERIALIEN
MATIÈRES POLYMÈRES

(30) Priority: 17.07.2008 GB 0813093
(43) Date of publication of application: 30.03.2011
(73) Proprietor: Invibio Limited, Lancashire FY5 4QD (GB)
(72) Inventor: JARMAN-SMITH, Marcus, Lancashire FY8 2EA (GB); WHITEHEAD, Tony, Lancashire FY6 8DW (GB); ELLERAY, Andrew, Lancashire BB5 2PF (GB); DEVINE, John, Lancashire FY6 7EE (GB)
(74) Representative: Brierley, Anthony Paul
(86) International application number: PCT/GB2009/050853
(87) International publication number: WO 2010/007424

(56) References cited:
- WO-A-95/04765
- WO-A-2007/051307
- US-A- 4 226 886
- US-A- 5 969 020

## Description

This invention relates to polymeric materials and particularly, although not exclusively, relates to porous polymeric materials for use, for example in making medical implants or parts thereof.

It is known from WO2007/051307 to make porous medical implants from polyetheretherketone. In the method described, a mixture of polyetheretherketone and salt (e.g. sodium chloride) is placed in a mould cavity, compressed and heated to melt the polyetheretherketone but not the salt and form a moulded part. After subsequent cooling to solidify the mixture, the moulded material is placed in a water bath at 100°C to dissolve the salt from the moulded part and define a porous moulded part.

Disadvantageously, the compression moulding process described is not suitable for making many types of parts and lacks versatility. For example, the process generally must use polymer powder to achieve good blending with the salt and is limited to stock shapes for subsequent machining or simple part designs. Additionally, there is a risk that parts made may be brittle due to the force used in compression and the stresses introduced. Furthermore, the parts may be subject to higher risks of contamination, risk of polymer degradation and of entrapment of gas.

It is an object of the present invention to address problems associated with making porous materials.

According to a first aspect of the invention, there is provided a mass of material comprising particles which include polymeric material and fugitive material.

The mass of material can be used in subsequent process steps to manufacture parts, for example medical implants or parts thereof, which are arranged to be porous. Further details are included hereinafter.

Said mass of material may include particles having a volume in the range 0.1 to 1ml, preferably in the range 0.3 to 0.8ml, more preferably in the range 0.4 to 0.8 ml. Preferably substantially all particles in the mass have a volume as aforesaid.

The average volume (total volume of particles in the mass of material divided by the total number of said particles) may be at least 0.1ml, preferably at least 0.3ml, more preferably at least 0.4ml. The average volume (as described) may be less than 0.8ml.

Said mass of material may include particles having a diameter of at least 1mm, preferably at least 2mm. The diameter may be less than 6mm, preferably less than 5mm, more preferably less than 4mm. Preferably, substantially all particles in the mass have diameters as aforesaid.

The average diameter (sum of diameters of all particles divided by the total number) of said particles may be at least 1 mm, preferably at least 2mm. The average diameter may be less than 6mm, preferably less than 5mm, more preferably less than 4mm.

Said mass of material may include particles having a weight in the range 0.01g to 0.1g, suitably in the range 0.02g to 0.08g, preferably in the range 0.03g to 0.06g. Preferably, substantially all particles in the mass have an average weight as aforesaid.

The average weight of particles in the mass of material (i.e. total weight of all particles divided by the total number) may be in the range 0.01g to 0.1g, suitably in the range 0.02g to 0.08g, preferably in the range 0.03g to 0.06g. Preferably, substantially all particles in the mass have an average weight as aforesaid.

Said particles are preferably pellets or granules.

Said mass of material may include at least 1 kg, preferably at least 5kg, of particles.

Said mass of material may include particles comprising 10 to 90wt%, suitably 20 to 80wt%, preferably 30 to 80wt%, more preferably 40 to 80wt% of fugitive material. The mass of material may, in some cases include 50 to 80wt%, 60 to 80wt% or even 70 to 80wt% of fugitive material.

Said mass of material may include 10 to 90wt%, suitably 20 to 80wt%, preferably 30 to 80wt%, more preferably 40 to 80wt% of fugitive material. The mass of material may, in some cases include 50 to 80wt%, 60 to 80wt% or even 70 to 80wt% of fugitive material.

Said mass of material preferably consists essentially of said polymeric material and fugitive material.

Said mass of material may include 10 to 90wt%, preferably 20 to 80wt%, more preferably 20 to 60wt%, of said polymeric material. In some cases, the mass of material may include 20 to 50wt%, 20 to 40wt% or 20 to 30wt% of said polymeric material.

The ratio of the weight of polymeric material to the weight of fugitive material in said mass of material may be at least 0.1, preferably at least 0.2. Said ratio may be less than 10, preferably 8 or less, more preferably 5 or less. In some cases, the ratio may be in the range 0.25 to 1.

Where the mass of material includes more than one type of polymeric material which is arranged to define a matrix within which fugitive material is dispersed, weights (or other quantities) of said polymeric material referred to herein may refer to the sum of the total weight of all polymeric materials which are arranged to define a said matrix. Preferably, however, weights (or other quantities) of polymeric materials arranged to define a matrix refer to the weight of a single polymeric material. Preferably, particles in said mass of material include a single polymeric material which is arranged to define a matrix within which fugitive material is dispersed.

Whereas the mass of material includes more than one fugitive material dispersed within polymeric material, weights (or other quantities) of said fugitive material referred to herein may refer to the sum of the total weight of all fugitive materials. Preferably, however, weights (or other quantities) of fugitive materials refer to the weight of a single fugitive material. Preferably, particles in said mass of material include a single fugitive material dispersed within polymeric material.

Preferably, at least 90wt%, more preferably at least 95wt%, especially about 100% of said mass of material is made up of a single polymeric material and fugitive material.

Said mass of material suitably includes homogenous particles comprising said polymeric material and fugitive material. Preferably, the fugitive material is dispersed and/or distributed throughout the polymeric material. Preferably, the fugitive material is arranged and distributed so that a large proportion of particles of fugitive material contact other particles of fugitive material - i.e. preferably a negligible number of particles of fugitive material are completely encased in said polymeric material. This may be achieved by using high levels of fugitive material and ensuring that polymeric material and fugitive material are fully mixed to produce a homogenous mass.

Preferably, a said particle in said mass of material includes fused polymeric material, for example fused particles of polymeric material. Said fused polymeric material suitably defines a network which is suitably substantially continuous throughout a said particle. Said network is suitably irregularly shaped. Fugitive material in a particle may be arranged between parts of and/or may contact said network. Fugitive material may comprise discrete particles which may contact one another but are preferably not fused to one another. Preferably, at least 80%, more preferably at least 90wt%, especially substantially all particles in said mass of material are as described.

Particles in said mass of material are preferably obtainable in a process which comprises melt processing, for example, extruding, polymeric material and fugitive material. An extrudate, for example in the form of a lace, may be cut for example chopped, to define particles

Said polymeric material preferably comprises a bio-compatible polymeric material. Said polymeric material preferably comprises a thermoplastic polymer. Said polymeric material may be bioabsorbable or non-bioabsorbable. Examples of bioabsorbable polymers include poly(dioxanone), polyglycolic acid, polylactic acid, polyalkylene oxalates, polyanhydrides and copolymers thereof. Examples of non-bioabsorbable polymers include polyurethanes, polyamides, polyesters, polyolefins, polyarylether sulphones and polyarylether ketones.

Said polymeric material may have a Notched Izod Impact Strength (specimen 80mm x 10mm x 4mm with a cut 0.25mm notch (Type A), tested at 23°C, in accordance with ISO180) of at least 4KJm⁻², preferably at least 5KJm⁻², more preferably at least 6KJm⁻². Said Notched Izod Impact Strength, measured as aforesaid, may be less than 10KJm⁻², suitably less than 8KJm⁻².

The Notched Izod Impact Strength, measured as aforesaid, may be at least 3KJm⁻², suitably at least 4KJm⁻², preferably at least 5KJm⁻². Said impact strength may be less than 50 KJm⁻², suitably less than 30KJm⁻².

Said polymeric material suitably has a melt viscosity (MV) of at least 0.06 kNsm⁻², preferably has a MV of at least 0.09 kNsm⁻², more preferably at least 0.12 kNsm⁻², especially at least 0.15 kNsm⁻².

MV is suitably measured using capillary rheometry operating at 400°C at a shear rate of 1000s⁻¹ using a tungsten carbide die, 0.5x3.175mm.

Said polymeric material may have a MV of less than 1.00 kNsm⁻², preferably less than 0.5 kNsm⁻².

Said polymeric material may have a MV in the range 0.09 to 0.5 kNsm⁻², preferably in the range 0.14 to 0.5 kNsm⁻², more preferably in the range 0.3 to 0.5 kNsm⁻².

Said polymeric material may have a tensile strength, measured in accordance with ISO527 (specimen type 1b) tested at 23°C at a rate of 50mm/minute of at least 20 MPa, preferably at least 60 MPa, more preferably at least 80 MPa. The tensile strength is preferably in the range 80-110 MPa, more preferably in the range 80-100 MPa.

Said polymeric material may have a flexural strength, measured in accordance with ISO178 (80mm x 10mm x 4mm specimen, tested in three-point-bend at 23°C at a rate of 2mm/minute) of at least 50 MPa, preferably at least 100 MPa, more preferably at least 145 MPa. The flexural strength is preferably in the range 145-180MPa, more preferably in the range 145-164 MPa.

Said polymeric material may have a flexural modulus, measured in accordance with ISO178 (80mm x 10mm x 4mm specimen, tested in three-point-bend at 23°C at a rate of 2mm/minute) of at least 1 GPa, suitably at least 2 GPa, preferably at least 3 GPa, more preferably at least 3.5 GPa. The flexural modulus is preferably in the range 3.5-4.5 GPa, more preferably in the range 3.5-4.1 GPa.

Said polymeric material may be amorphous or semi-crystalline. It is preferably semi-crystalline.

The level and extent of crystallinity in a polymer is preferably measured by wide angle X-ray diffraction (also referred to as Wide Angle X-ray Scattering or WAXS), for example as described by Blundell and Osborn (Polymer 24, 953, 1983). Alternatively, crystallinity may be assessed by Differential Scanning Calorimetry (DSC).

The level of crystallinity of said polymeric material may be at least 1%, suitably at least 3%, preferably at least 5% and more preferably at least 10%. In especially preferred embodiments, the crystallinity may be greater than 25%.

The main peak of the melting endotherm (Tm) of said polymeric material (if crystalline) may be at least 300°C.

Said polymeric material includes a repeat unit of general formula 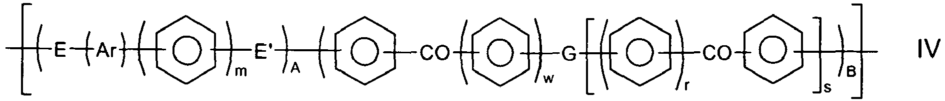 or a repeat unit of general formula 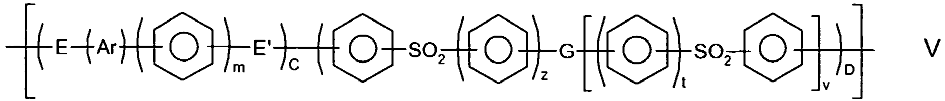 wherein A, B, C and D independently represent 0 or 1, E and E' independently represent an oxygen or a sulphur atom or a direct link, G represents an oxygen or sulphur atom, a direct link or a -O-Ph-O- moiety where Ph represents a phenyl group, m, r, s, t, v, w, and z represent zero or 1 and Ar is selected from one of the following moieties (i) to (v) which is bonded via one or more of its phenyl moieties to adjacent moieties
(i) 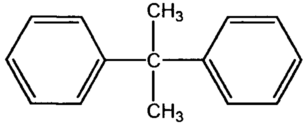
(ii) 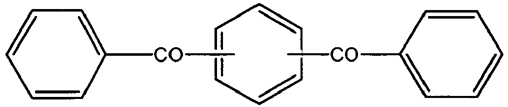
(iii) 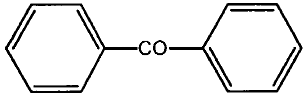
(iv) 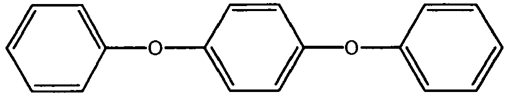
(v)

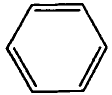

Unless otherwise stated in this specification, a phenyl moiety has 1,4-, linkages to moieties to which it is bonded.

Said polymeric material may be a homopolymer which includes a repeat unit of IV or V or may be a random or block copolymer of at least two different units of IV and/or V.

As an alternative to a polymeric material comprising units IV and/or V discussed above, said polymeric material may include a repeat unit of general formula 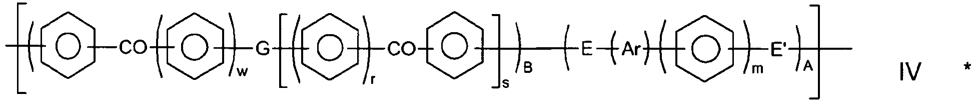 or a homopolymer having a repeat unit of general formula 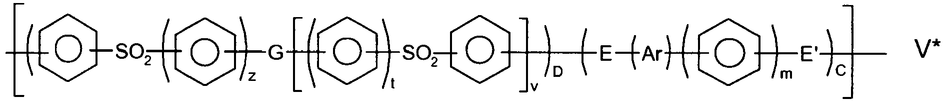 wherein A, B, C, and D independently represent 0 or 1 and E, E', G, Ar, m, r, s, t, v, w and z are as described in any statement herein.

Said polymeric material may be a homopolymer which includes a repeat unit of IV* or V* or a random or block copolymer of at least two different units of IV* and/or V*.

Preferably, said polymeric material is a homopolymer having a repeat unit of general formula IV.

Preferably Ar is selected from the following moieties (vi) to (x)
(vi) 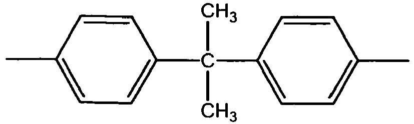
(vii) 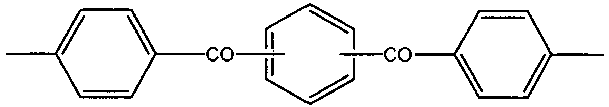
(viii) 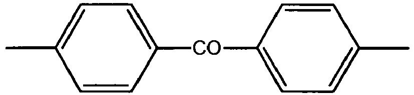
(ix) 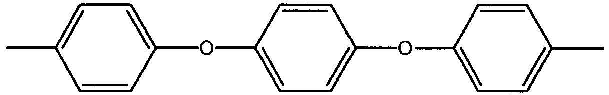
(x)

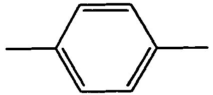

In (vii), the middle phenyl may be 1,4- or 1,3-substituted. It is preferably 1,4-substituted.

Suitable moieties Ar are moieties (ii), (iii), (iv) and (v) and, of these, moieties, (ii), (iii) and (v) are preferred. Other preferred moieties Ar are moieties (vii), (viii), (ix) and (x) and, of these, moieties (vii), (viii) and (x) are especially preferred.

An especially preferred class of polymeric materials are polymers (or copolymers) which consist essentially of phenyl moieties in conjunction with ketone and/or ether moieties. That is, in the preferred class, the polymer material does not include repeat units which include -S-, -SO₂- or aromatic groups other than phenyl. Preferred bio-compatible polymeric materials of the type described include:
(a) a polymer consisting essentially of units of formula IV wherein Ar represents moiety (v), E and E' represent oxygen atoms, m represents 0, w represents 1, G represents a direct link, s represents 0, and A and B represent 1 (i.e. polyetheretherketone).
(b) a polymer consisting essentially of units of formula IV wherein E represents an oxygen atom, E' represents a direct link, Ar represents a moiety of structure (ii), m represents 0, A represents 1, B represents 0 (i.e. polyetherketone);
(c) a polymer consisting essentially of units of formula IV wherein E represents an oxygen atom, Ar represents moiety (ii), m represents 0, E' represents a direct link, A represents 1, B represents 0, (i.e. polyetherketoneketone).
(d) a polymer consisting essentially of units of formula IV wherein Ar represents moiety (ii), E and E' represent oxygen atoms, G represents a direct link, m represents 0, w represents 1, r represents 0, s represents 1 and A and B represent 1. (i.e. polyetherketoneetherketoneketone).
(e) a polymer consisting essentially of units of formula IV, wherein Ar represents moiety (v), E and E' represents oxygen atoms, G represents a direct link, m represents 0, w represents 0, s, r, A and B represent 1 (i.e. polyetheretherketoneketone).
(f) a polymer comprising units of formula IV, wherein Ar represents moiety (v), E and E' represent oxygen atoms, m represents 1, w represents 1, A represents 1, B represents 1, r and s represent 0 and G represents a direct link (i.e. polyetherdiphenyl-ether-phenyl-ketone-phenyl-).

Said polymeric material may consist essentially of one of units (a) to (f) defined above. Alternatively, said polymeric material may comprise a copolymer comprising at least two units selected from (a) to (f) defined above. Preferred copolymers include units (a). For example, a copolymer may comprise units (a) and (f); or may comprise units (a) and (e).

Said polymeric material preferably comprises, more preferably consists essentially of, a repeat unit of formula (XX) 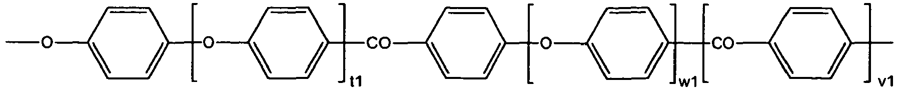 where t1, and w1 independently represent 0 or 1 and v1 represents 0, 1 or 2. Preferred polymeric materials have a said repeat unit wherein t1=1, v1=0 and w1=0; t1=0, v1=0 and w1=0; t1=0, w1=1, v1=2; or t1=0, v1=1 and w1=0. More preferred have t1=1, v1=0 and w1=0; or t1=0, v1=0 and w1=0. The most preferred has t1=1, v1=0 and w1=0.

In preferred embodiments, said polymeric material is selected from polyetheretherketone, polyetherketone, polyetherketoneetherketoneketone and polyetherketoneketone. In a more preferred embodiment, said polymeric material is selected from polyetherketone and polyetheretherketone. In an especially preferred embodiment, said polymeric material is polyetheretherketone.

Said fugitive material suitably refers to any material which can be combined with the polymeric material and particles formed, but can subsequently be removed from association with the polymeric material.

Said fugitive material may have a melting point which is greater than the melting point of said polymeric material. The melting point of the fugitive material may be at least 100°C, suitably at least 200°C, preferably at least 300°C, more preferably at least 350°C greater than the melting point of said polymeric material. The melting point of the fugitive material may be at least 450°C, preferably at least 500°C, more preferably at least 600°C, especially at least 700°C.

In some embodiments, where said polymeric material has a low melting point (e.g. 40°C), the fugitive material could be a biological material, for example collagen chips. Such a fugitive material may be removed in a biological reaction, for example by enzyme digestion.

Said mass of material may include discrete particles of fugitive material which are suitably dispersed in the polymeric material. Said fugitive material dispersed in particles in said mass of material may have a D₅₀ in the range 1 to 20000µm. Preferably, the D₅₀ is in the range 10 to 2000µm. In some embodiments wherein, for example, the mass of material is to be used to produce a porous member to be used in an osseoconductive capacity, the D₅₀ may be in the range 10 to 1200µm to allow pores to be produced which are suitable for bone ingrowth. In other embodiments, lower porosity may be required in which case the D₅₀ may be in the range 10 to 100µm.

In some cases, fugitive material may be in a fibrous form. However, it is preferred for the fugitive material to be in a particulate form.

Said fugitive material may be arranged to be dissolved away from the polymeric material in a subsequent process step or may be arranged to be reacted to allow its removal from the polymeric material. Preferably, a fugitive material is selected which can be solubilised by a solvent under conditions which do not dissolve to any significant degree said polymeric material.

Said fugitive material is preferably arranged to be dissolved away from the polymeric material in a subsequent process step using a biologically safe solvent. Thus, if any solvent residue remains in a medical implant made from the mass of material, the residue will not be significantly detrimental to a patient associated with the implant. In some cases, fugitive material may be arranged to be removed by means of acids or bases. For example, if calcium carbonate is used as a fugitive material, hydrochloric acid could be used as a solvent; starch/sugar fugitive materials could be removed with dilute sulphuric acid; and silica gel fugitive materials could be removed with sodium hydroxide.

Said fugitive material may have a solubility in water of at least 5g/100ml, suitably at least 15g/100ml, preferably at least 20g/100ml, more preferably at least 25g/100ml, especially at least 30g/100ml, wherein in each case solubility is measured at 25°C.

In some embodiments, said fugitive material or part of said fugitive material may be arranged to be leached from an implant when the implant is in situ in a human body. In this case, said fugitive material or part of said fugitive material suitably has a water solubility as described above and, additionally, is preferably arranged to liberate a material which is non-toxic and/or not detrimental in vivo. The fugitive material or said part is preferably arranged to have a beneficial effect when liberated. For example, dissolution of said fugitive material or part of said fugitive material may liberate an anti-bacterial agent (e.g. silver or anti-biotic containing), a radioactive compound (e.g. which emits alpha, beta or gamma radiation for therapy, research, tracing, imaging, synovectomy or microdosimetry) or an active agent which may facilitate bone integration or other processes associated with bone (e.g. the active agent may be calcium phosphate).

When particles described are used in non-medical applications, fugitive materials may include colourants, dyes, lubricants or other active materials which are liberated in use to produce a desired effect.

Said fugitive material may be organic or inorganic. It is preferably inorganic.

Said fugitive material is preferably non-toxic.

Said fugitive material may comprise a polysaccharide, protein, polymer other than said polymeric material or other non-toxic materials which are soluble in a solvent which does not dissolve said polymeric material

Said fugitive material may comprise a salt, metal oxide or glass.

Said fugitive material is preferably water-soluble. It may be selected from sugar, sodium chloride, Na₂CO₃.10H₂O, sodium benzoate, sodium acetate, sodium nitrate, sodium tartrate, sodium citrate and magnesium sulphate including hydrated forms of any of the aforesaid. Preferably, said fugitive material is a salt, more preferably a water-soluble salt, especially a water soluble sodium salt. Sodium chloride is preferred.

Said particles may include other additives. Such other additives preferably have a melting point which is greater than the melt-processing temperature of said polymeric material, for example the melting point. Preferably, such other additives have a decomposition temperature which is greater, preferably by at least 50°C, than the melting point of the polymeric material.

Other additives may be bio-active. Examples include hydroxyapatite and bioglasses. Said other additives may be fugitive materials as described above which are arranged to be leached from an implant when the implant is in situ in a human body and the aforementioned description of such fugitive materials applies to said other additives described here *mutatis mutandis.* Thus, bio-active additives may be arranged to leach in use from a component, for example an implantable component, which may be made from the mass of material or may be arranged to facilitate integration of human tissue (e.g. bone) into an implantable component.

Other additives may comprise reinforcing agents and may comprise additives which are arranged to improve mechanical properties of components made from the mass of material. Preferred reinforcing agents comprise fibres.

Said fibres may comprise a fibrous filler or a non-fibrous filler. Said fibres may include both a fibrous filler and a non-fibrous filler.

A said fibrous filler may be continuous or discontinuous. In preferred embodiments a said fibrous filler is discontinuous.

Preferably, fibres which are discontinuous have an average length of less than 10mm, preferably less than 7mm.

A said fibrous filler may be selected from inorganic fibrous materials, high-melting organic fibrous materials and carbon fibre.

A said fibrous filler may be selected from inorganic fibrous materials, non-melting and high-melting organic fibrous materials, such as aramid fibres, and carbon fibre.

A said fibrous filler may be selected from glass fiber, carbon fibre, asbestos fiber, silica fiber, alumina fiber, zirconia fiber, boron nitride fiber, silicon nitride fiber, boron fiber, fluorocarbon resin fibre and potassium titanate fiber. Preferred fibrous fillers are glass fibre and carbon fibre.

A fibrous filler may comprise nanofibres.

A said non-fibrous filler may be selected from mica, silica, talc, alumina, kaolin, calcium sulfate, calcium carbonate, titanium oxide, ferrite, clay, glass powder, zinc oxide, nickel carbonate, iron oxide, quartz powder, magnesium carbonate, fluorocarbon resin and barium sulfate. The list of non-fibrous fillers may further include graphite, carbon powder and nanotubes. The non-fibrous fillers may be introduced in the form of powder or flaky particles.

Preferred reinforcing agents are glass fibre and/or carbon fibre.

Other additives may comprise radiopacifiers, for example barium sulphate and any other radiopacifiers described in co-pending application PCT/GB2006/003947. Up to 20wt%, or up to 5wt% of radiopacifiers may be included. Preferably, less than 1wt%, more preferably no radiopacifier is included.

Other additives may include colourants, for example titanium dioxide. Up to 3wt% of colourant may be included but preferably less than 1wt%, more preferably no, colourant is included.

Said mass of material may include up to 15wt%, for example up to 10wt% of other materials -that is, in addition to said polymeric material and fugitive material. Thus in one preferred embodiment, said mass of material includes 20 to 80wt% of fugitive material (preferably of a single type of fugitive material), 20 to 80wt% of a polymeric material (preferably of a single type of polymeric material) and up to 15wt% of other materials, for example of the type described. In another preferred embodiment, said mass of material includes 40 to 80wt% of fugitive material (preferably of a single type of fugitive material), 20 to 60wt% of a polymeric material (preferably of a single type of polymeric material) and up to 10wt% of other materials, for example of the type described. In a further preferred embodiment, said mass of material includes 55 to 80wt% of fugitive material (preferably of a single type of fugitive material), 20 to 45wt% of a polymeric material (preferably of a single type of polymeric material) and up to 5wt% of other materials, for example of the type described.

Preferably, said particles (more preferably said mass of material) consists essentially of polymeric material and fugitive material and more preferably consists essentially of a single type of polymeric material and a single type of fugitive material.

According to a second aspect of the invention, there is provided a method of making a mass of material according to the first aspect, the method comprising:
(a) melt-processing a mixture comprising polymeric material and fugitive material; and
(b) forming said mixture into particles.

The mass of material, particles, polymeric material and fugitive material may have any feature described in said first aspect.

At the end of step (a), the mixture is preferably substantially homogenous. The method of the second aspect may include a step (a)* prior to step (a) which comprises forming said mixture. The method may involve selecting polymeric material and fugitive material and contacting them. Initial contact may occur at ambient temperature; for example polymeric material and fugitive material may be dry mixed. Alternatively and preferably, fugitive material may be initially contacted with polymeric material at above ambient temperature for example when the polymeric material is molten. In a preferred embodiment, fugitive material is initially contacted with polymeric material in a compounder for example in the screw of a compounder.

When said particles include other additives as described according to said first aspect, said other additives may be included in the mixture melt processed in step (a) and may be formed in step (a)* It is preferred that additives are selected which can withstand the processing conditions used in the method of the second aspect.

It is preferred that ingredients in said mixture are dried prior to preparation of the mixture, particularly when the mixture includes a salt or any other potentially corrosive ingredient, thereby to reduce corrosion of any apparatus used in the method.

Melt-processing of the mixture may be undertaken in an extruder. Thus, polymeric material and fugitive material may be mixed and/or melt processed in an extruder. The polymeric material and fugitive material may be melt processed to define particles by extruding a length of mixture and comminuting said length, for example by cutting, chopping or the like, to define particles of the type described. Such particles suitably comprise fused polymeric material which is suitably defined by polymeric material melted in the melt-processing such that said polymeric material suitably defines a network; and fugitive material arranged within the network, wherein said fugitive material is not melted by said melt-processing.

The mixture is suitably melt-processed to define said particles described which are suitably then cooled.

According to a third aspect of the invention, there is provided a method of making a component, the method comprising:
(a) selecting a mass of material comprising particles which include polymeric material and fugitive material;
(b) melt-processing said mass of material to define at least a part of the component; and
(c) optionally, removing the fugitive material.

Said mass of material may be as described according to the first aspect and/or be made in a method according to the second aspect.

The method may be used in non-medical or medical applications. Non-medical applications include manufacture of filters, meshes, light weight parts and parts arranged to elute lubricants.

The component may comprise a part or the whole of a device which may be incorporated into or associated with a human body. Thus, the component may suitably be a part of or the whole of a medical implant. The medical implant may be arranged to replace or supplement soft or hard tissue. It may replace or supplement bone. It may be used in addressing trauma injury or craniomaxillofacial injury. It may be used in joint replacement, for example as part of a hip or finger joint replacement; or in spinal surgery.

In the method, said mass of material is suitably melt processed at a temperature above the melting temperature of polymeric material in said mass of material but at a temperature which is less than the melting temperature of the fugitive material.

Said mass of material is preferably melt processed in an extruder or moulder, for example injection moulder. Extrusion or moulding may be used to directly produce said component (or part thereof); or may be used to produce a precursor of said component (or part thereof) which may be subjected to further processing, for example machining, to define said component (or part thereof).

When said mass of material is melt processed in an extruder, a fibre, rod, tube, bar, plate or film may be produced. A rod, tube, bar or plate may define a precursor of a said component (or part thereof) which may be further processed for example by machining. A said film may itself be used directly or may be associated with other materials to define a device. References to extrusion include co-extrusion to define components which include regions of different compositions and/or properties.

A said component may include a hollow or void region.

When said mass of material is melt processed in a moulder any desired shape may be produced. Near net-shaped ingots may be produced for further processing, for example machining; or a component which does not require any significant machining prior to use may be produced. An injection moulder is a preferred moulder. References to moulding include overmoulding to define components which include regions of different compositions and/or properties.

A particularly advantageous method may comprise making a component (or part thereof) which includes regions of different compositions. For example, a first region may be made by moulding a said mass of material of the type described; and a second region may be made by moulding a second region adjacent the first region, wherein said second region is moulded from a second composition which is different to the composition of said mass of material. Said second composition may be in the form of a mass of material as described according to the first aspect (e.g. it includes polymeric material and fugitive material) or may not be a mass of material in accordance with the first aspect (e.g. it may not include a fugitive material). In a preferred embodiment, said first and second regions may comprise the same polymeric material, for example polyetheretheketone. The regions may differ on the basis of the amount or identity of fugitive material used in the preparation of said regions. The component (or part thereof) may include one or a plurality of further regions.

The method may be used to produce a device for promoting fusion of first and second vertebrae, the device comprising;
a first solid region formed of non-porous polyetheretherketone and
a first porous region including a porous polyetheretherketone architecture,
wherein the first porous region is bonded to the first solid region. Such a device may be as described in US2008/0161927.

In another method, a component (or part thereof) may be made which includes regions of different porosities or regions which include different levels of fugitive material (and may later define regions of different porosities). For example, a first region may be made by moulding a said mass of material of the type described which includes a first amount of fugitive material; and a second region may be made by moulding a said mass of material of the type described which includes a second amount of fugitive material, wherein said first and second amounts are different. The component may include one or a plurality of further regions. Thus, the method may be used to produce a component (or part thereof) which includes different levels of fugitive material (or porosity if the fugitive material is removed). For example, a component (or part thereof) may include gradually increasing or stepped levels of fugitive material (or porosity) on moving from one position to another position.

In a first embodiment, a component (or part thereof) made in the method may be used, for example as a part or the whole of a device which may be incorporated into or associated with a human body, only after fugitive material has been removed in step (c) of the method. In this case, the method may be used to define a component (or part thereof) which is porous prior to use. The fugitive materials sole purpose in the method may be to facilitate such pore formation.

In a second embodiment, a component (or part thereof) made in the method may be arranged to be used, for example as a part or the whole of a device which may be incorporated into or associated with a human body, whilst fugitive material remains associated with the device and/or prior to any removal or fugitive material. Thus, the fugitive material is suitably of a type which has no detrimental effects when present in a human body. Preferably, such a fugitive material is arranged to leach out of the component (or part thereof) in vivo. The material leaching out is suitably an active ingredient which may have a positive effect within the body. Suitably, leaching of said fugitive material is arranged to produce increasing levels of porosity in said component (or part thereof) in vivo. Such porosity may also be arranged to have a positive effect.

In a third embodiment, a component (or part thereof) made in the method may be treated to remove its fugitive material as described in accordance with the first embodiment. Thereafter, porous regions of the component (or part thereof) may be impregnated with another material. Such a material may be arranged to leach from the component (or part thereof) in vivo or may be arranged to remain within pores in the component (or part thereof) and exert an effect, for example a biological effect, when present. An example of a material which may be impregnated as aforesaid is collagen or a drug loaded bio-absorbable polymer.

In a fourth embodiment, a component (or part thereof) of the first, second or third embodiments may include a hollow or void region which may be impregnated with another material as described in the third embodiment.

When said method includes removing said fugitive material in step (c), the method suitably involves contacting a product formed after melt-processing in step (b) with a means for removing the fugitive material, suitably so as to define porosity. Contact may take place at any time. However, contact suitably takes place after any machining or physical manipulation of said product that may be involved in making a component (or part thereof) for use, for example as part of or the whole of a device which may be incorporated into or associated with a human body. This is because a product may have more strength to withstand, for example machining, whilst fugitive material is in situ.

Said means for removing the fugitive material may be arranged to solubilise said fugitive material. Said means suitably comprises a solvent. Said solvent preferably comprises water and more preferably includes at least 80wt%, preferably at least 95wt%, especially at least 99wt% water. The solvent preferably consists essentially of water.

Means for removing the fugitive material may comprise contacting the product formed after melt processing with a solvent formulation (preferably comprising water as aforesaid) which is at a temperature of greater than 100°C and a pressure above ambient pressure thereby to charge the solvent formulation with fugitive material and separating the charged solvent from the product.

In the method, said solvent formulation may be at a temperature of greater than 150°C, suitably greater than 200°C when contacted with said product. Said solvent formulation may be at a temperature of less than 500°C, suitably less than 450°C, preferably less than 400°C, more preferably less than 350°C when contacted with said product.

The solvent formulation may be under a pressure of at least 4 bar, suitably at least 8 bar, preferably at least 10 bar when contacted with said product. The pressure may be less than 300 bar, preferably less than 200 bar, more preferably less than 100 bar, especially less than 50 bar. The pressure is preferably selected to maintain the solvent formulation in the liquid state when in contact with said product.

Preferably, in the method, the solvent formulation is arranged to flow from a first region to a third region via a second region in which said product is arranged.

According to a fourth aspect, there is provided a component or a part thereof obtainable in the method of the third aspect.

According to a fifth aspect, there is provided a component or a part thereof per se.

Any feature of any aspect of any invention or embodiment described herein may be combined with any feature of any aspect of any invention or embodiment described herein *mutatis mutandis.*

Specific embodiments of the invention will now be described, by way of example, with reference to the accompanying drawings, in which:
Figures 1a and 1b are a perspective view and a cross-section respectively of an acetabular cup with an overmoulded porous layer;
Figure 2a and 2b are a perspective view and a cross-section respectively of an alternative acetabular cup with an overmoulded area of porous material of one type and an adjacent overmoulded area of another material;
Figure 3 is a schematic representation of an implantable device comprising areas having different properties.

In the figures, the same or similar parts are annotated with the same reference numerals.

### Example 1 - Preparing granules

Prior to compounding, the raw materials -unfilled (polyetheretherketone) PEEK polymer with medium viscosity (Invibio LT2 obtained from Invibio Limited, UK) and pharmaceutical grade sodium chloride (obtained from Sigma) - were prepared. The sodium chloride was sorted to an appropriate particle size suited to give pores for osseoconductivity (particle range 100-1000 µm diameter). This was achieved through sieving through graduated meshes. To aid the removal of atmospheric water and benefit processing, 70kg of the sodium chloride was placed in a drying oven for 5 hours at 200°C. This was repeated for 30kg PEEK to remove the 0.5% of water which PEEK absorbs.

A twin-screw compounder was used, fitted with a strand die and suitable polymer and powder metering equipment. The sodium chloride and PEEK raw materials were hand charged to two compounder hoppers. At the output end a strand conveyer, a pelletiser, a classifier to separate longs and a suitable clean collection bin were positioned. An appropriate size of machine was chosen to reduce excessive polymer residence time especially since sodium chloride may also heat and retain heat. The sodium chloride was fed in at a ratio of 70wt% to 30wt% PEEK, determined to facilitate interconnectivity of pores in use. The addition of the sodium chloride to the PEEK polymer occurred when the polymer was in a fluid state (due to shear and temperature generated in the screw). A lower viscosity PEEK polymer (medium viscosity LT2) was selected to help counteract the increase in viscosity as a result of the addition of high quantities of filler. The twin-screw compounder ran at a temperature between 360-400°C. A normal screw profile fabricated from stainless steel was used with a minimum L/D ratio of 45:1. At the extrusion end a twin hole die with a 4mm orifice was used. The temperature profile along the screw varied between 360-400°C.

The main screw rotation speed was 150-250 rpm (but could be higher for highly loaded materials). It was maintained within the former range to avoid long residence times and potential polymer degradation. The throughput rate was 10kg/hr (with potential for up to 20dk/hr). The compounded material containing 70wt% sodium chloride in 30wt% PEEK was extruded as a continual lace of approximately 3mm. This was air cooled as it was captured onto a strand conveyer. To convert laces to granule pellets a pelletiser was used with a classifier to separate longs and collection was into a suitable clean bin.

### Example 2 - Injection moulding of granules into near net shape ingots

Ingots of dimensions 20mm x 20mm x 100mm, suitably for machining, were made in an injection moulding machine using granules of Example 1.

### Example 3 - injection moulding of granules into plaques

The procedure described in Example 2 was followed to prepare 150mm x 75mm x 10mm plaques which may be machined into representative samples for medical devices which may benefit from porosity.

### Example 4 - Formation of partially porous regions

Near net shaped ingots of dimensions 20mm x 20mm x 100mm having partially porous regions were made. Within a mould tool cavity was placed some preformed ingots, manufactured using the technique described in Example 2, but subsequently machined to remove a predetermined volume. These were half ingots and other partial ingots. These half and partial ingots consisted of PEEK with dispersed sodium chloride and were solid ingots that had been machined to remove sufficient volume to permit the flow of new polymer into the remaining mould cavity space when the ingots were re-inserted into the mould tool. Unfilled PEEK was charged into the injection moulding machine reading for injection into the remaining cavity space. The procedure of Example 2 was generally used to produce moulded ingots which were ejected from the mould and allowed to cool prior to annealing. The resultant half and partial ingots consisted of 20mm x 20mm x 100mm ingots with various proportions of solid PEEK and sodium chloride loaded PEEK regions. Ingots were machined into representative samples of medical devices which may benefit from porosity. Porosity was then produced in sodium chloride loaded PEEK regions by leaching of sodium chloride as described hereinafter.

### Example 5- Extrusion into rod stock

Rod stock was made using the granules of Example 1 by extrusion. The rod stock could be machined to define a medical device and subsequently porosity produced by removal of the sodium chloride as described hereinafter.

### Example 6 - Formation of film

To create film and thin sheets of material from fugitive granules a template consisting of several parts sandwiched together was used. To the underside of a metal plate with a central shape cut-out (typically a square) and of 1mm thickness was placed a thin aluminium foil sheet which was coated with non-stick agent to facilitate release. Into the central cut-out was placed sufficient granules from Example 1 to cover roughly the cut-out area and to overfill the tool to allow for shrinkage.

### Example 7 - Large scale film production

Film may be prepared by extruding granules through a slit die to define film of desired dimensions.

### Example 8 - Injection moulding into direct device or component shape

By following the methods described above, granule of Example 1 may be fed to an injection moulding machine with mould cavities arranged to define a part or the whole of a medical device without machining.

### Example 9 - Removing "skin" from parts to be leached

In some cases, a "skin" may be formed on the outside of a part produced, for example by injection moulding. Whilst when a part is machined prior to leaching of fugitive material, such machining itself may result in removal of the skin, in other cases, for example where a direct device or shape is produced as described in Example 8, it may be desirable to remove the skin prior to dissolution, to facilitate penetration of solvent into the part. To this end, micro-ablative blasting (e.g. a MICROBLASTER (Trade Mark)) may be used with sodium carbonate as an abrasive medium.

### Example 10 - General procedure for removal of fugitive material

Purification apparatus as described in PCT/GB02/02525 may be used. The apparatus includes a pressure vessel which has a heated and thermally insulated jacket. Upstream of the vessel is a water supply line for delivering pressurized water into the vessel and downstream of the vessel there is a water drain for removing water to waste. In use, a sample of material (e.g. machined ingot, plaque or rod; or film or injection moulded part) is placed in the vessel and then liquid water at high pressure and temperature is caused to flow through the vessel. The water penetrates the PEEK polymer and dissolves the fugitive material.

The processes and/or products described in the aforementioned examples may have wide scale applications.

In general terms the granules of Example 1 may be used in any situation where standard granules comprising filled or unfilled polyetheretherketone may be used. These include extrusion, co-extrusion, moulding or overmoulding processes.

Films may be prepared with a defined range of porosity to replace soft or hard tissues. Such an arrangement may be of utility in treatment of trauma or craniomaxillofacial injury where a thin supporting layer may be required for structural reinforcement. The provision of pores in the material facilitates tissue anchorage and integration through in-growth. Laminates could be made comprising layers of varying thicknesses or compositions which may be porous, non-porous or partially porous. For example, a 0.4mm porous film with 200µm interconnected pores could be layered upon a PEEK film containing 30wt% barium sulphate. These layers could be un-joined/un-bonded or may be fused using adhesives (silicone, epoxy or other implantable adhesive) or through melt welding, laser welding, ultrasonic welding or other means. Additional layers could be incorporated throughout or on the underside. This flexibility has the benefit of tailored thickness versatility according to patient specific (or other) requirements. For example, a porous upper film with a middle radioopaque film, with a film containing a fugitive material that resorbs in vivo and which also resorbs to dose out an active (eg. drug, growth factor, anti-microbial) could be provided. The (porous or non porous) layers could also be further modified to encourage more rapid ingrowth of tissue into the pores, for example by possessing a surface modification to improve bone ingrowth (eg. coating, plasma, growth factor or stimulatory protein attached via surface chemistry modification or peptide linkages).

Tubing may be made using granules comprising fugitive material at a suitable loading and of appropriate particle sizes, by melting the granules and extruding material through a dye to form a tube shape. This shape can be cooled (eg. in air on a conveyor line), cut into lengths as required and the fugitive material removed as described in Example 10. The tubes can be made rigid or thin walled depending on the proposed application and may have applications as components, or as functional parts. The lumen of the tube could remain empty, be filled with unfilled, or filled PEEK, or another polymer, or metal. This additional material in the lumen can be inserted and permanently bonded using melt processes (eg. over extrusion onto a material). The internal material may contain factors that pass out through the pores to convey a particular activity (eg. internal controllable degradation material such as a resorbable polymer containing drug) within a structural porous PEEK tube of pore sizes in the range 1 to 1000µm depending on application.

By way of example, small diameter tubing/hollow fibres possessing microporosity may be made for use as a bone ingrowth fibre using fugitive material (eg. tricalcium phosphate (TCP). This fugitive could resorb in-vivo to leave pores for cell ingrowth. Whilst resorbing, the fibre material may have the beneficial effect of possessing bone mineralization factors. The fugitive material could be any inert or stimulatory material of diameters specific to purpose, application or cell type. A hollow fibre could have pores refilled with a material more amenable for the desired purpose. For example, a hollow fibre may be made by leaching sodium chloride from an extruded fibre, and then dipping the porous fibre in collagen to fill in pores and resorb in-vivo.

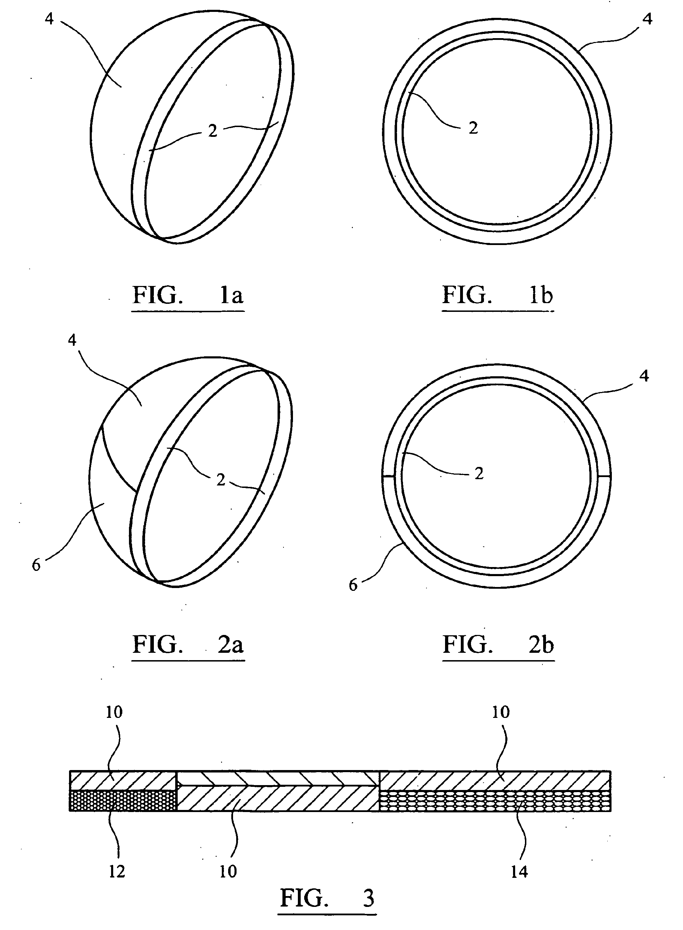

In another embodiment, screws or bolts used in medical applications in vivo may be overmoulded using the granules of figure 1 to allow insertion but, by defining specific porous regions, tissue ingrowth may be improved.

Mono or multi-filament fibres may be made by extrusion using the granules of Example 1 and subsequent leaching as described in Example 10. The porosity may afford benefits for integration or loading of factors into or onto the fibres. Fibres may be used for sutures or yarns that could be subsequently woven or braided or knitted or non-woven into textiles suitable for implantation. The fugitive material used may need to be of small diameter to pass through filtration units typically required for fibre production of polymers. For example a fugitive material and/or filler could be mixed with PEEK with a particle size of 1 to 100 microns depending on the target mono filament or multifilament diameter. For example, a monofilament of 100 microns could possess a fugitive filler (eg. TCP of 10µm). Additionally the fibre could include fillers suitable to confer other implantable benefits (eg. radiopacity through barium sulfate or other safe filler, reinforcement through nano fibres or glass or carbon or bioglass fibres, or over extruded to possess a core of another material( eg. core of steel, or tantalum overmoulded with PEEK filled with fugitive and/or other filler(s)). Such fibres could be made into textiles using a mix of different fibre yarns with different properties.

Granules as described in Example 1 may be used in the manufacture of devices requiring better fixation or tissue integration. Referring to figure 1, a polymeric acetabular cup includes a cup body 2 which may be made from a composite material comprising PEEK and carbon fibre and an overmoulded outer layer 4 comprising a polymer, for example PEEK containing a bioactive ingredient (e.g. hydroxyapatite, tricalcium phosphate or a bioglass) which may be overmoulded from granules made as described in Example 1. The bioactive ingredient may leach from layer 4 in vivo leaving a porous layer 4 which may facilitate tissue integration. As an alternative, an acetabular cup may include a PEEK/sodium chloride outer layer 4, and the sodium chloride may be leached out as described in Example 10, before implantation.

The layer 4 could be further modified to encourage more rapid ingrowth of tissue for example bone into the pores, for example by being surface modified (e.g. by coating, use of a plasma, growth factor or stimulatory protein attached via surface chemistry modification or peptide linkages) improve bone ingrowth.

In a further variation illustrated in figures 2a and 2b, an acetabular cup includes cup body 2 and an outer layer 4 which this time only covers a hemispherical surface. The other hemispherical surface 6 may be formed of a different material (e.g. PEEK with an alternative filler or mouldable material such as an elastomer).

A spinal cage implant may be fabricated including from selected areas of unfilled and porous material. For example, a cage may include a thin outer "halo" rim of a composite comprising PEEK and carbon fibre with porous PEEK in the middle; or an unfilled PEEK outer may be provided over a porous centre; or an unfilled PEEK outer may be provided over a centre region comprising hydroxyapatite filled PEEK.

In general terms, granules as described may be used in conjunction with other materials to provide combination materials with targeted functional areas to allow devices to be made which may confer beneficial properties in particular regions. For example, referring to figure 3, regions 10 may comprise an unfilled or carbon fibre filled PEEK frame arranged to provide the main structural support, closely mimicking bone. Certain areas, for example in regions 12, 14 which may be at an end or on one particular side/area and which may come into contact with bone and require ingrowth, may be moulded to define a porous PEEK or comprise PEEK filled with material enhancing bone integration (e.g. hydroxyapatite or TCP which may be used as a fugitive material which could be leached prior to implantation or may resorb during implantation to leave pores). If a region is porous prior to implantation, it could be enhanced by surface modification or loading with degradable material that confers additional benefits such as a drug, anti-infection agent or stimulatory factor.

A craniomaxillofacial implant may be designed with porous regions to prevent implant migration through tissue ingrowth and a smooth area to facilitate overlying muscle movement (e.g. jaw). The porous area may be part of all of one side or a combination of two sides.

Devices which may include combination materials may include finger joints or craniomaxillofacial devices.

In each case referred to, porous materials may be further coated or treated in selected regions, for example by plasma treatment, hydroxyapatite coating or any non line-of-sight method may be used to promote osseointegration or cell ingrowth. Pores may be coated or filled with another degradable material (e.g. PLGA, LCP) to give additional initial mechanical strength, encourage ingrowth or be loaded with a stimulatory factor (e.g. growth factor or chemical or drug or drug immobilized within a resorbable material such as PLGHA or LCP) that may elute or release over time.

## Claims

1. A mass of material comprising particles which include polymeric material and fugitive material, wherein said polymeric material includes a repeat unit of general formula 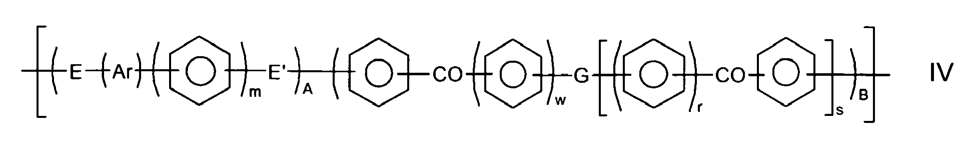 or a repeat unit of general formula 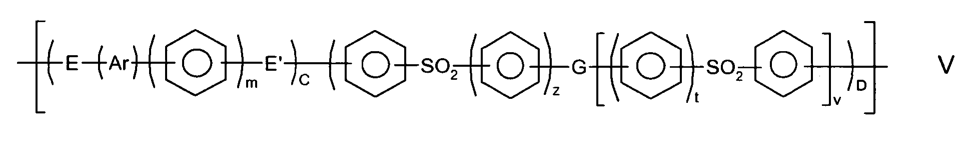 wherein A, B, C and D independently represent 0 or 1, E and E' independently represent an oxygen or a sulphur atom or a direct link, G represents an oxygen or sulphur atom, a direct link or a -O-Ph-O- moiety where Ph represents a phenyl group, m, r, s, t, v, w, and z represent zero or 1 and Ar is selected from one of the following moieties (i) to (v) which is bonded via one or more of its phenyl moieties to adjacent moieties
(i) 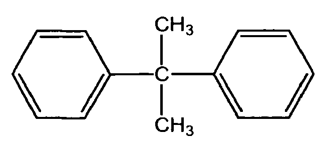
(ii) 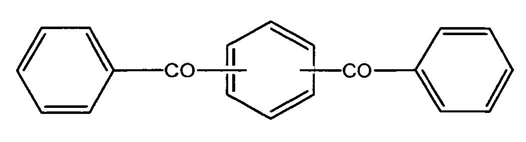
(iii) 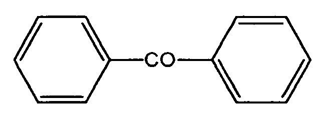
(iv) 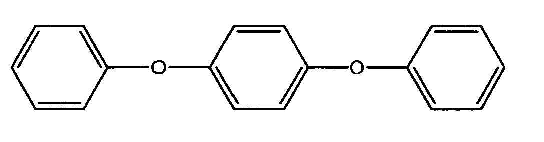
(v)

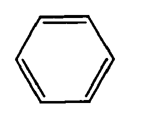

2. A mass according to claim 1, which includes particles having a volume in the range 0.1 to 1ml, and, optionally, the average volume of said particles is at least 0.1ml and is less than 0.8ml.

3. A mass according to claim 1 or claim 2, wherein the average weight of particles in the mass of material is in the range 0.01g to 0.1 g.

4. A mass according to any preceding claim, which includes particles comprising 40 to 80wt% of fugitive material and 20 to 60wt% of said polymeric material.

5. A mass of material according to any preceding claim, wherein a said particle in said mass of material includes fused polymeric material.

6. A mass according to any preceding claim, wherein said polymeric material comprises a repeat unit of formula (XX) 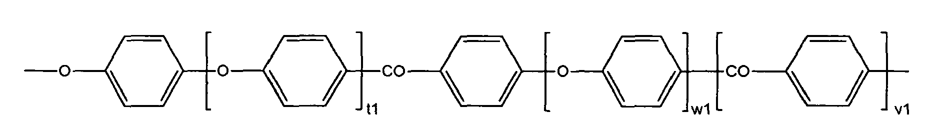 where t1, and w1 independently represent 0 or 1 and v1 represents 0, 1 or 2.

7. A mass according to claim 6, wherein t1=1, v1=0 and w1=0.

8. A mass according to any preceding claim, wherein said fugitive material dispersed in particles in said mass of material has a D₅₀ in the range 1 to 20000µm.

9. A mass according to any preceding claim, wherein said fugitive material comprises a salt, metal oxide or glass.

10. A mass according to any preceding claim which comprises polyetheretherketone and sodium chloride and said mass is in the form of pellets or granules having a volume in the range 0.1 to 1ml and the average weight of particles in the mass of material is in the range 0.01g to 0.1g.

11. A method of making a mass of material according to any of claims 1 to 10, the method comprising:
(a) melt-processing a mixture comprising polymeric material and fugitive material; and
(b) forming said mixture into particles.

12. A method according to claim 11, wherein melt-processing of the mixture is undertaken in an extruder.

13. A method of making a component, the method comprising:
(a) selecting a mass of material comprising particles which include polymeric material and fugitive material, according to any of claims 1 to 10;
(b) melt-processing said mass of material to define at least a part of the component; and
(c) optionally, removing the fugitive material.

14. A method according to claim 13, which comprises making a component (or part thereof) which includes regions of different compositions, wherein a first region is made by moulding a mass of material as described in any of claims 1 to 10; and a second region is made by moulding a second region adjacent the first region, wherein said second region is moulded from a second composition which is different to the composition of said mass of material.

15. A method according to claim 13 or claim 14, wherein when said method includes removing said fugitive material in step (c), the method comprises contacting a product formed after melt-processing in step (b) with a means for removing the fugitive material, so as to define porosity.

## Patentansprüche

1. Materialmasse, umfassend Teilchen, die polymeres Material und fugitives Material enthalten, wobei das polymere Material eine Wiederholungseinheit der allgemeinen Formel 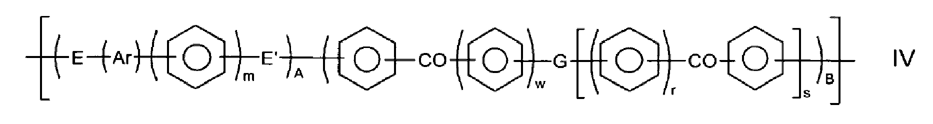 oder eine Wiederholungseinheit der allgemeinen Formel 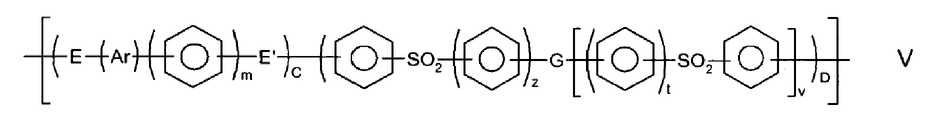 enthält, wobei A, B, C und D unabhängig für 0 oder 1 stehen, E und E' unabhängig für ein Sauerstoff - oder Schwefelatom oder eine direkte Verknüpfung stehen, G für ein Sauerstoff- oder Schwefelatom, eine direkte Verknüpfung oder eine -O-Ph-O-Gruppierung, wobei Ph eine Phenylgruppe bedeutet, steht, m, r, s, t, v, w und z für null oder 1 stehen und Ar unter einer der folgenden Gruppierungen (i) bis (v), die über eine oder mehrere ihrer Phenylgruppierungen an benachbarte Gruppierungen gebunden ist, ausgewählt ist:
(i) 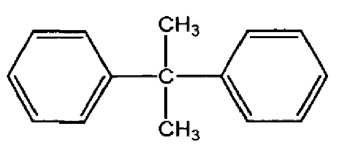
(ii) 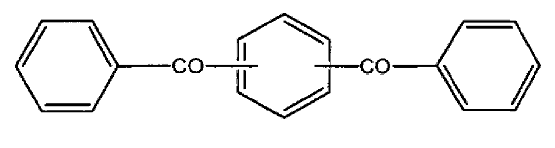
(iii) 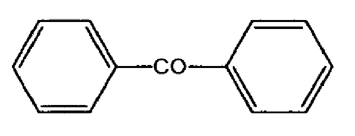
(iv) 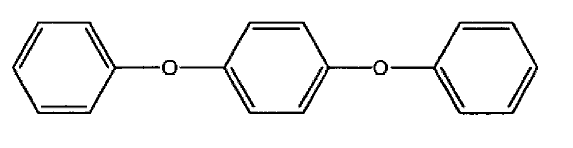
(v)

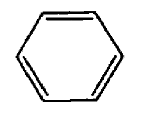

2. Masse nach Anspruch 1, die Teilchen mit einem Volumen im Bereich von 0,1 bis 1 ml enthält, wobei das durchschnittliche Volumen der Teilchen gegebenenfalls mindestens 0,1 ml und weniger als 0,8 ml beträgt.

3. Masse nach Anspruch 1 oder Anspruch 2, wobei das durchschnittliche Gewicht von Teilchen in der Materialmasse im Bereich von 0,01 g bis 0,1 g liegt.

4. Masse nach einem der vorhergehenden Ansprüche, die Teilchen mit 40 bis 80 Gew.-% fugitivem Material und 20 bis 60 Gew.-% des polymeren Materials enthält.

5. Masse nach einem der vorhergehenden Ansprüche, wobei ein besagtes Teilchen in der Materialmasse verschmolzenes polymeres Material enthält.

6. Masse nach einem der vorhergehenden Ansprüche, wobei das polymere Material eine Wiederholungseinheit der Formel (XX) 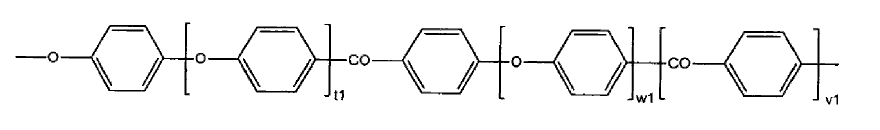 umfasst, wobei t1 und w1 unabhängig für 0 oder 1 stehen und v1 für 0, 1 oder 2 steht.

7. Masse nach Anspruch 6, wobei t1 - 1, v1 - 0 und w1 = 0.

8. Masse nach einem der vorhergehenden Ansprüche, wobei das in Teilchen in der Materialmasse dispergierte fugitive Material einen D₅₀-Wert im Bereich von 1 bis 20.000 µm aufweist.

9. Masse nach einem der vorhergehenden Ansprüche, wobei das fugitive Material ein Salz, Metalloxid oder Glas umfasst.

10. Masse nach einem der vorhergehenden Ansprüche, die Polyetheretherketon und Natriumchlorid umfasst und in Form von Pellets oder Granulat mit einem Volumen im Bereich von 0,1 bis 1 ml vorliegt, wobei das durchschnittliche Gewicht von Teilchen in der Materialmasse im Bereich von 0,01 g bis 0,1 g liegt.

11. Verfahren zur Herstellung einer Materialmasse nach einem der Ansprüche 1 bis 10, bei dem man:
(a) eine Mischung, die polymeres Material und fugitives Material umfasst, in der Schmelze verarbeitet und
(b) die Mischung zu Teilchen formt.

12. Verfahren nach Anspruch 11, bei dem die Verarbeitung der Mischung in der Schmelze in einem Extruder erfolgt.

13. Verfahren zur Herstellung einer Komponente, bei dem man:
(a) eine Materialmasse, umfassend Teilchen, die polymeres Material und fugitives Material enthalten, nach einem der Ansprüche 1 bis 10 auswählt;
(b) die Materialmasse in der Schmelze verarbeitet, um mindestens einen Teil der Komponente zu definieren; und
(c) gegebenenfalls das fugitive Material entfernt.

14. Verfahren nach Anspruch 13, bei dem man eine Komponente (oder einen Teil davon), der Bereiche unterschiedlicher Zusammensetzungen umfasst, herstellt, wobei ein erster Bereich durch Formen einer Materialmasse gemäß einem der Ansprüche 1 bis 10 hergestellt wird und ein zweiter Bereich durch Formen eines zweiten Bereichs, der an den ersten Bereich angrenzt, hergestellt wird, wobei der zweite Bereich aus einer zweiten Zusammensetzung, die sich von der Zusammensetzung der Materialmasse unterscheidet, geformt wird.

15. Verfahren nach Anspruch 13 oder Anspruch 14, bei dem man das fugitive Material in Schritt (c) entfernt, wobei ein nach der Verarbeitung in der Schmelze in Schritt (b) gebildetes Produkt mit einem Mittel zur Entfernung des fugitiven Materials in Berührung gebracht wird, um Porosität zu definieren.

## Revendications

1. Masse de matériau comprenant des particules qui comprennent un matériau polymère et un matériau fugitif, ledit matériau polymère comprenant un motif de répétition de formule générale 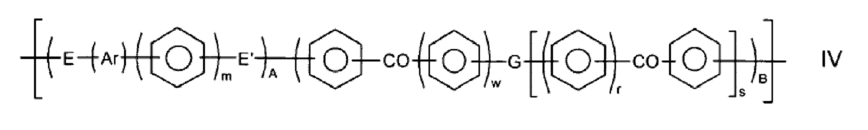 ou un motif de répétition de formule générale 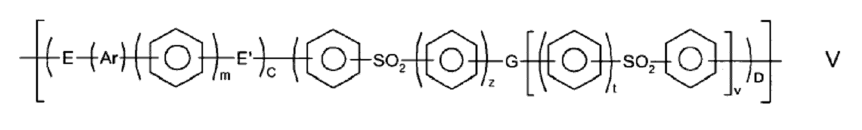 où A, B, C et D représentent indépendamment 0 ou 1, E et E' représentent indépendamment un atome d'oxygène ou de soufre ou une liaison directe, G représente un atome d'oxygène ou de soufre, une liaison directe ou un fragment -O-Ph-O- où Ph représente un groupe phényle, m, r, s, t, v, w et z représentent zéro ou 1 et Ar est choisi parmi un des fragments suivants (i) à (v) qui est lié via un ou plusieurs de ses fragments phényle à des fragments adjacents.
(i) 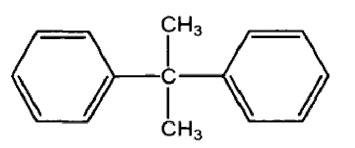
(ii) 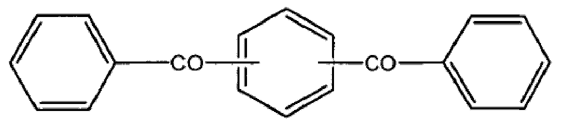
(iii) 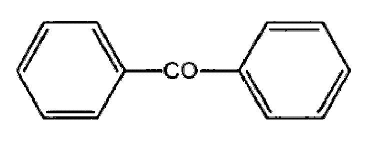
(iv) 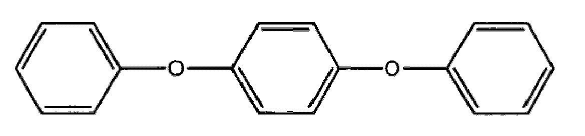
(v)

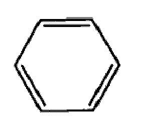

2. Masse selon la revendication 1, qui comprend des particules ayant un volume dans la plage de 0,1 à 1 ml, et, facultativement, le volume moyen desdites particules est d'au moins 0,1 ml et est inférieur à 0,8 ml.

3. Masse selon la revendication 1 ou la revendication 2, dans laquelle le poids moléculaire des particules dans la masse de matériau est dans la plage de 0,01 g à 0,1 g.

4. Masse selon l'une quelconque des revendications précédentes, qui comprend des particules comprenant de 40 à 80 % en poids de matériau fugitif et de 20 à 60 % en poids dudit matériau polymère.

5. Masse de matériau selon l'une quelconque des revendications précédentes, dans laquelle une desdites particules dans ladite masse de matériau comprend un matériau polymère condensé.

6. Masse selon l'une quelconque des revendications précédentes, dans laquelle ledit matériau polymère comprend un motif de répétition de formule (XX) 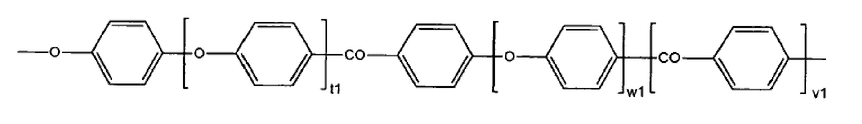 dans laquelle t1, et w1 représentent indépendamment 0 ou 1 et v1 représente 0, 1 ou 2.

7. Masse selon la revendication 6, dans laquelle t1 = 1, v1 = 0 et w1 = 0.

8. Masse selon l'une quelconque des revendications précédentes, dans laquelle ledit matériau fugitif dispersé dans lesdites particules dans ladite masse de matériau a un D₅₀ dans la plage de 1 à 20000 µm.

9. Masse selon l'une quelconque des revendications précédentes, dans laquelle ledit matériau fugitif comprend un sel, un oxyde de métal ou un verre.

10. Masse selon l'une quelconque des revendications précédentes qui comprend une polyétheréthercétone et du chlorure de sodium et ladite masse sous la forme de pastilles ou de granules ayant un volume dans la plage de 0,1 à 1 ml et le poids moyen de particules dans la masse de matériau est dans la plage de 0,01 g à 0,1 g.

11. Procédé de fabrication d'une masse de matériau selon l'une quelconque des revendications 1 à 10, le procédé comprenant :
(a) le traitement à l'état fondu d'un mélange comprenant un matériau polymère et un matériau fugitif ; et
(b) la formation dudit mélange en particules.

12. Procédé selon la revendication 11, dans lequel le traitement à l'état fondu du mélange est effectué dans une extrudeuse.

13. Procédé de fabrication d'un composant, le procédé comprenant :
(a) la sélection d'une masse de matériau comprenant des particules qui comprennent un matériau polymère et un matériau fugitif, selon l'une quelconque des revendications 1 à 10 ;
(b) le traitement à l'état fondu de ladite masse de matériau pour définir au moins une partie du composant ; et
(c) facultativement, le retrait du matériau fugitif.

14. Procédé selon la revendication 13, qui comprend la fabrication d'un composant (ou une partie de celui-ci) qui comprend des régions de différentes compositions, une première région étant fabriquée par moulage d'une masse de matériau telle que décrite dans l'une quelconque des revendications 1 à 10 ; et une deuxième région étant fabriquée par moulage d'un deuxième région adjacente à la première région, ladite deuxième région étant moulée à partir d'une deuxième composition qui est différente de la composition de ladite masse de matériau.

15. Procédé selon la revendication 13 ou la revendication 14, dans lequel, lorsque ledit procédé comprend le retrait du matériau fugitif dans l'étape (c), le procédé comprend la mise en contact d'un produit formé après traitement à l'état fondu dans l'étape (b) avec des moyens pour éliminer le matériau fugitif, de manière à définir une porosité.
